# EUROPÄISCHE PATENTANMELDUNG

(11) **EP 0 565 969 A1**
(43) Veröffentlichungstag der Anmeldung: **20.10.1993**
(21) Anmeldenummer: 93105475.3
(22) Anmeldetag: 02.04.1993
(51) Int. Cl.: C07D 307/94, C07D 311/94, C07D 307/93, C07B 57/00

(54) **Tricyclische Lactole, ihre Verwendung als Racematspaltungsmittel und Verfahren zu ihrer Herstellung**

(30) Priorität: 13.04.1992 AT 769/92
(71) Anmelder: DSM Chemie Linz GmbH, 4021 Linz (AT)
(72) Erfinder: Noe, Christian, Dr., W-6000 Frankfurt am Main II (DE); Gmeiner, Günter, Dr., A-2500 Baden (AT)
(74) Vertreter: Kunz, Ekkehard, Dr.

(57) **Zusammenfassung**

(R)- und (S)-Enantiomere der Formel 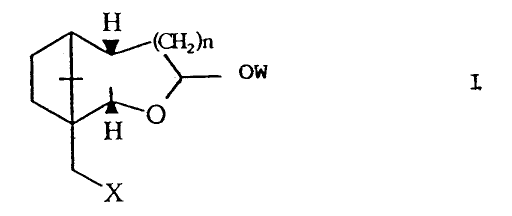
in der n 1 oder 2,
W Wasserstoff, Alkyl, Cycloalkyl oder den Rest 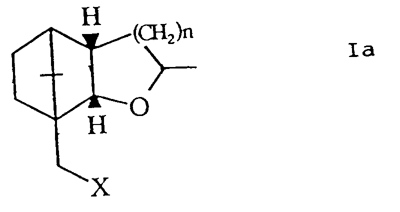
bedeuten, sowie für den Fall, daß W Wasserstoff ist, deren Anhydroverbindungen der Formel 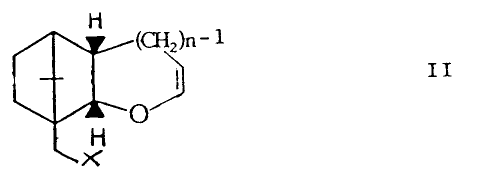
dadurch gekennzeichnet, daß X Halogen, SO₃H, SO₂Cl oder SO₂NR₁R₂ bedeutet, wobei R₁ und R₂ unabhängig voneinander Wasserstoff, substituiertes oder unsubstituiertes, verzweigtes oder unverzweigtes Alkyl, substituiertes oder unsubstituiertes Aryl oder Heteroaryl bedeuten, oder R₁ und R₂ gemeinsam mit dem Stickstoff einen substituierten oder unsubstituierten Heterocyclus bilden, sowie ein Verfahren zu ihrer Herstellung und ihre Verwendung zur Razemattrennung.

## Beschreibung

Die vorliegende Erfindung betrifft Reagentien zur Razemattrennung, welche mit deutlicher Bevorzugung mit einem der Enantiomeren eines razemischen Gemisches reagieren, Verfahren zu ihrer Herstellung und deren Verwendung.

Aus US-PS 4 497 960 sind (R)- und (S)-Enantiomere, in welchen ein 5 oder 6 gliedriger Lactolring in cis-Konfiguration an einem Bornanring anelliert ist, sowie deren Anhydroverbindungen bekannt, die als acetalische Schutzgruppen und als Reagentien zur Razemattrennung und asymmetrischen Induktion eingesetzt werden können.
Diese Verbindungen reagieren mit alkoholischen und Säure-Hydroxylgruppen, mit Amino- oder Mercaptogruppen von razemischen Verbindungen häufig mit einer vom Verhältnis 1 : 1 abweichenden Selektivität. Das Ausmaß dieser Selektivität ist jedoch in der Regel so gering, daß der Effekt nur in beschränktem Umfang bei Razemattrennungen Vorteile bietet.

Gegenstand der vorliegenden Erfindung sind (R)- und (S)-Enantiomere der Formel 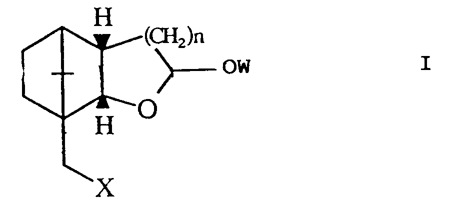
in der n 1 oder 2,
W Wasserstoff, Alkyl, Cycloalkyl oder den Rest 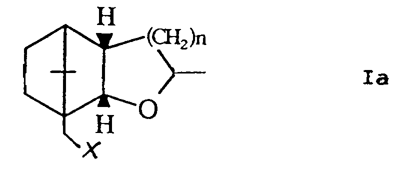
bedeuten, sowie für den Fall, daß W Wasserstoff ist, deren Anhydroverbindungen der Formel 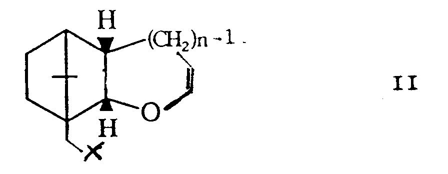
in der n wie oben definiert ist, die dadurch gekennzeichnet sind, daß X Halogen, SO₃H, SO₂Cl oder SO₂NR₁R₂ bedeutet, wobei R₁ und R₂ unabhängig voneinander Wasserstoff, substituiertes oder unsubstituiertes, verzweigtes oder unverzweigtes Alkyl, substituiertes oder unsubstituiertes Aryl oder Heteroaryl bedeuten, oder R₁ und R₂ gemeinsam mit dem Stickstoff einen substituierten oder unsubstituierten Heterocyclus bilden.

Dabei bedeuten W Wasserstoff, den Rest der Formel Ia
oder einen Alkyl- oder Cycloalkylrest, bevorzugt mit 1 - 20 C-Atomen, besonders bevorzugt mit 1 - 10 C-Atomen, der gegebenenfalls substituiert sein kann, etwa durch Hydroxy, COOR₃ oder Amino.
Der Alkyl- und der Cycloalkylrest können dabei verzweigt oder unverzweigt sein. Beispiele für Alkyl- bzw. Cycloalkylreste sind Methyl, Ethyl, n-Propyl, i-Propyl, Cyclopropyl, n-Butyl, sec. Butyl, tert. Butyl, Cyclobutyl, Pentyl, Cyclopentyl, Hexyl, Cyclohexyl, Heptyl, Cycloheptyl, Decyl, Cyclodecyl, Dodecyl und Octadecyl. R₃ kann dabei Wasserstoff oder (C₁ - C₆) Alkyl bedeuten.

X bedeutet Halogen, wie Brom, Chlor, Fluor und Jod, bevorzugt Brom, weiters SO₃H, SO₂Cl oder die Gruppe SO₂NR₁R₂. R₁ und R₂ können dabei unabhängig voneinander Wasserstoff oder ein Alkylrest sein, bevorzugt mit 1 - 20 C-Atomen, besonders bevorzugt mit 1 - 10 C-Atomen, der gegebenenfalls substituiert sein kann, beispielsweise durch Hydroxy, Amino, COOR₃, CONR₄R₅ oder Aryl, wie beispielsweise Phenyl. Der Alkylrest kann dabei verzweigt oder unverzweigt sein. R₁ und R₂ können auch einen Arylrest mit bevorzugt 6 - 10 C-Atomen, wie beispielsweise ein Phenyl- oder Naphthylrest, der gegebenenfalls etwa durch Hydroxy, COOR₃, Amino oder (C₁ - C₆)Alkyl substituiert sein kann, bedeuten. Weiters können sie ein Heteroarylrest, bevorzugt 5 oder 6 gliedrig, mit O, N oder S als Heteroatom, sein. R₃ hat dabei obige Bedeutung. R₄ und R₅ können unabhängig voneinander Wasserstoff, (C₁ - C₆)Alkyl, Phenyl oder Benzyl sein. Beispiele für einen Heteroarylrest sind Pyrrolyl, Furanyl, Thiophenyl, Pyridinyl, Pyrazolyl, Isoxazolyl, Isothiazolyl, Imidazolyl, Pyridinyl, Pyridazinyl, Pyrimidinyl und Pyrazinyl. R₁ und R₂ können jedoch auch gemeinsam mit dem Stickstoffatom einen 3- bis 7gliedrigen Heterocyclus, bevorzugt einen 5- oder 6 gliedrigen, bilden, der gegebenenfalls durch COOR₃ substituiert sein kann, wobei R₃ obige Bedeutung hat. Beispiele sind Aziridinyl, Pyrrolidinyl, Piperidinyl, Prolin oder Prolinmethyl- oder ethylester.

Bevorzugte Verbindungen der Formeln I und II sind solche, in denen W Wasserstoff oder der Rest der Formel Ia ist und X die Gruppe SO₂NR₁R₂ bedeutet, wobei R₁ und R₂ unabhängig voneinander Wasserstoff oder ein verzweigtes oder unverzweigtes, unsubstituiertes oder mit COOR₃, CONR₄R₅ oder Phenyl substituiertes (C₁ - C₁₀)Alkyl sein kann, oder wobei R₁ und R₂ gemeinsam mit dem Stickstoffatom einen 5 oder 6 gliedrigen Heterocyclus der gegebenenfalls durch COOR₃ substituiert ist, bilden. R₃ ist dabei Wasserstoff oder (C₁-C₆)Alkyl, R₄ und R₅ können unabhängig voneinander Wasserstoff, (C₁ - C₆)Alkyl, oder Benzyl sein.

Besonders bevorzugte Einzelverbindungen sind:
1. (2alpha,3a-alpha,4beta,7alpha,7a-alpha)-7-Brommethyl-8,8-dimethyl-octahydro-4,7-methanobenzofuran-2-ol
2. (2alpha,3a-alpha,4beta,7alpha,7a-alpha)-2-Octahydro-2-hydroxy-8,8-dimethyl-4,7-methanobenzofuran-7-yl-methansulfonsäure
3. (2alpha(2'R*,3'aS*,4'S*,7'R*,7'aS*),3a-alpha,4beta,7alpha,7a-alpha)-2,2'-Oxybis((octahydro-8,8-dimethyl-4,7-methanobenzofuran-7-yl)-methansulfon-säurechlorid
4. (2alpha,3a-alpha,4beta,7alpha,7a-alpha)-N,N-Dimethyl-(octahydro-2-hydroxy-8,8-dimethyl-4,7-methanobenzofuran-7-yl)-methansulfonamid
5. (2alpha,3a-alpha,4beta,7alpha,7a-alpha)-N,N-Diethyl-(octahydro-2-hydroxy-8,8-dimethyl-4,7-methanobenzofuran-7-yl)-methansulfonamid
6. (2alpha,3a-alpha,4beta,7alpha,7a-alpha)-N,N-Bis-(phenylmethyl)-(octahydro-2-hydroxy-8,8-dimethyl-4,7-methanobenzofuran-7-yl)methansulfonamid
7. (2alpha(2'R*,3'aS*,4'S*,7'R*,7'aS*),3a-alpha,4beta,7alpha,7a-alpha)-2,2'-Oxybis(N-((octahydro-8,8-dimethyl-4,7-methanobenzofuran-7-yl)methylsulfonyl)-N-(phenylmethyl)-glycin)
8. (2alpha(2'R*,3'aS*,4'S*,7'R*,7'aS*),3a-alpha,4beta,7alpha,7a-alpha)-2,2'-Oxybis(1-((octahydro-8,8-dimethyl-4,7-methanobenzofuran-7-yl)methylsulfonyl)-L-prolinmethylester)
9. (2alpha(2'R*,3'aS*,4'S*,7'R*,7'aS*),3a-alpha,4beta,7alpha,7a-alpha)-2,2'-Oxybis(1-((octahydro-8,8-dimethyl-4,7-methanobenzofuran-7-yl)methylsulfonyl)-L-prolin)
10. (2alpha(2'R*,3'aS*,4'S*,7'R*,7'aS*),3a-alpha,4beta,7alpha,7a-alpha)-2,2'-Oxybis(1-((octahydro-8,8-dimethyl-4,7-methanobenzofuran-7-yl)methylsulfonyl)-D-prolinmethylester)
11. (2a(2'R*,3'aS*,4'S*,7'R*,7'aS*),3a-alpha,4beta,7alpha,7a-alpha)-2,2'-Oxybis(1-((octahydro-8,8-dimethyl-4,7-methanobenzofuran-7-yl)methylsulfonyl)-D-prolin)
12. (2alpha(2'R*,3'aS*,4'S*,7'R*,7'aS*),3a-alpha,4beta,7alpha,7a-alpha)-2,2'-Oxybis(N-((octahydro-8,8-dimethyl-4,7-methanobenzofuran-7-yl)methylsulfonyl)-N-(2-methoxy-2-oxoethyl)-glycinmethylester)
13. (2alpha(2'R*,3'aS*,4'S*,7'R*,7'aS*),3a-alpha,4beta,7alpha,7a-alpha)-2,2'-Oxybis(N-(carboxymethyl)-N-((octahydro-8,8-dimethyl-4,7-methanobenzofuran-7-yl)methylsulfonyl)-glycin)
14. (2alpha(2'R*,3'aS*,4'S*,7'R*,7'aS*),3a-alpha,4beta,7alpha,7a-alpha)-2,2'-Oxybis((octahydro-8,8-dimethyl-4,7-methanobenzofuran-7-yl)methylsulfonyl)-beta-alaninmethylester)
15. (2alpha(2'R*,3'aS*,4'S*,7'R*,7'aS*),3a-alpha,4beta,7alpha,7a-alpha)-2-2'-Oxybis((octahydro-8,8-dimethyl-4,7-methanobenzofuran-7-yl)methylsulfonyl)-beta-alanin)
16. (2alpha(2'R*,3'aS*,4'S*,7'R*,7'aS*),3a-alpha,4beta,7alpha,7a-alpha)-2,2'-Oxybis((octahydro-8,8-dimethyl-4,7-methanobenzofuran-7-yl)methylsulfonyl)-N-(phenylmethyl)-beta-alaninmethylester)
17. (2alpha(2'R*,3'aS*,4'S*,7'R*,7'aS*),3a-alpha,4beta,7alpha,7a-alpha))-2,2'-Oxybis((octahydro-8,8-dimethyl-4,7-methanobenzofuran-7-yl)methylsulfonyl)-N-(phenylmethyl)-beta-alanin)
18. (2a(2'R*,3'aS*,4'S*,7'R*,7'aS*),3a-alpha,4beta,7alpha,7a-alpha)-2,2'-Oxybis(2-(N-((octahydro-8,8-dimethyl-4,7-methanobenzofuran-7-yl)methylsulfonyl)-N-((phenylmethylamino)oxoethyl)-amino)-acetamid)
19. (2alpha(2'R*,3'aS*,4'S*,7'R*,7'aS*),3a-alpha,4beta,7alpha,7a-alpha)-2,2'-Oxybis(1-(1-((octahydro-8,8-dimethyl-4,7-methanobenzofuran-7-yl)methylsulfonyl)-L-prolyl)-L-prolinmethylester
Alle Verbindungen der Formel I oder II können entweder als (R)- oder als (S)-Enantiomeres vorliegen.

Ein weiterer Gegenstand der vorliegenden Erfindung ist ein Verfahren zur Herstellung von Verbindungen der Formel I, das dadurch gekennzeichnet ist, daß
a) ein Lacton der Formel 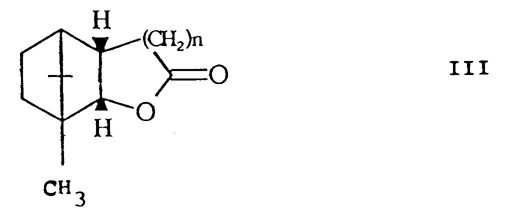 in der n wie oben definiert ist, mit einem Halogenierungsmittel oder mit Schwefelsäure und gegebenenfalls anschließender Überführung in das Sulfochlorid zu einem Lacton der Formel 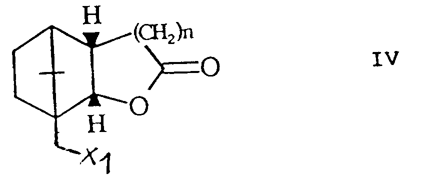 in der X₁ Halogen, SO₃H oder SO₂Cl bedeutet und n wie oben definiert ist, umgesetzt wird, worauf
b) das Lacton der Formel IV zu einer Verbindung der Formel V 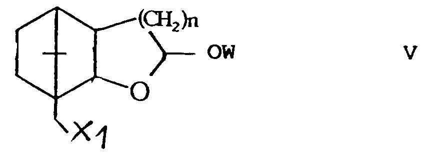 in der W, n und X₁ wie oben definiert sind,
   reduziert wird und
c) gegebenenfalls die Verbindung der Formel V für den Fall, daß X₁ die Gruppe SO₂Cl bedeutet, mit einem Amin der Formel

   NHR₁R₂ VI

   in der R₁ und R₂ wie oben definiert sind, zu einer Verbindung der Formel I umgesetzt wird oder
d) das Lacton der Formel IV im Fall, daß X₁ die Gruppe SO₂Cl bedeutet, gegebenenfalls zuerst mit einem Amin der Formel VI zu einer Verbindung der Formel 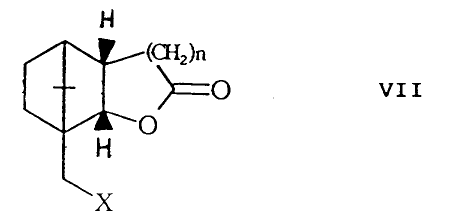 in der X und n wie oben definiert sind,
   umgesetzt wird und anschließend
e) die Verbindung der Formel VII zu einer Verbindung der Formel I reduziert wird.

Das als Ausgangsprodukt dienende Lacton der Formel III kann beispielsweise nach US 4 497 960 hergestellt werden.
Das Lacton kann erfindungsgemäß im 1. Schritt mit einem Halogenierungsmittel, etwa mit Brom in Gegenwart von rotem Phosphor, umgesetzt werden.
Dazu werden vorzugsweise das Lacton und der Phosphor vorgelegt und unter ständigem Rühren und unter Kühlung, bevorzugt unter Eiskühlung, Brom zugetropft. Nach dem Abklingen der dabei auftretenden Reaktion wird das Reaktionsgemisch auf etwa 50 - 90°C, bevorzugt 65 -75°C erhitzt, nochmals etwas Brom zugegeben, die Temperatur leicht etwa um 10°C erhöht und 2 bis 4 Stunden auf dieser Temperatur gehalten.
Anschließend wird der dabei entstehende Bromwasserstoff durch Spülen mit Inertgas, beispielsweise mit Stickstoff aus dem Reaktionsgemisch entfernt. Nach Zugabe geringer Mengen Wasser, etwa 1 - 5 ml wird das Reaktionsgemisch im Wasserstrahlvakuum bei etwa 120 - 150°C, bevorzugt 130 - 140°C abgesaugt.

Die Isolierung des Lactons der Formel IV erfolgt vorzugsweise mittels Extraktion und anschließendem Umkristallisieren.

Soll die SO₃H oder SO₂Cl-Gruppe eingeführt werden, wird das Lacton der Formel III sulfuriert oder sulfochloriert. Dazu wird beispielsweise das Lacton unter Kühlen mit konzentierter Schwefelsäure versetzt, sodaß die Temperatur 20°C nicht übersteigt. Dann wird das Reaktionsgemisch solange bei Raumtemperatur stehengelassen, bis die Reaktion beendet ist. Nach etwa 5 - 7 Tagen wird das entstandene Methansulfonsäurelacton abgesaugt und gewaschen. Gewünschtenfalls kann das Methansulfonsäurelacton in das Sulfochlorid, etwa mittels Phosphorpentachlorid oder anderen üblichen Reagenzien, überführt werden.

Ein nach einer der oben beschriebenen Möglichkeiten erhaltenes Lacton der Formel IV kann dann zuerst zu einem Lactol der Formel V reduziert werden.

Als Reduktionsmittel eignen sich beispielsweise Hydridverbindungen wie etwa Diisobutylaluminiumhydrid (DiBAl).
Das Lacton wird dazu gelöst, beispielsweise in einem wasserfreien Ether oder in CH₂Cl₂ und unter Intergasatmosphäre, beispielsweise N₂-Atmosphäre, dann wird bevorzugt eine DiBAl-Lösung, beispielsweise in Toluol, zugetropft, wobei die Temperatur etwa -80° bis -10°C beträgt. Das so erhaltene Lactol der Formel V wird dann nach etwa 0,5 bis 2 Stunden vorzugsweise mittels Extraktion gewonnen.

Um die selbstkondensierten Verbindungen der Formel I in der W den Rest der Formel Ia bedeutet, zu erhalten, wird bei der Extraktion dem Extraktionsmittel etwa eine katalytische Menge einer starken Säure, etwa 2nHCl zugegeben.

Bevorzugt können Lactole der Formel V in denen X₁ die Gruppe SO₂Cl ist mit einem Amin der Formel VI umgesetzt werden. Dazu wird das entsprechende Methansulfonsäurechloridlactol gelöst, etwa in absolutem Ether oder absolutem CH₂Cl₂, mit 2 - 3 Äquivalenten des entsprechenden Amins versetzt, und die entstandene Verbindung der Formel I abfiltriert und etwa durch Extraktion und Umkristallisieren gereinigt.
Geeignete Amine sind beispielsweise Dimethylamin, Diethylamin, Dibenzylamin, Phenylmethylglycin, Prolinmethylester, Prolin, (N-(2-Methoxy-2-oxoethyl)glycinmethylester), (N-(2-Carboxyethyl)glycin), Alaninmethylester, Alanin, N-(Phenylmethyl)alaninmethylester, N-(Phenylmethyl)alanin, Leucin, N-((Phenylmethylamino)oxoethyl)-amino)-acetamid oder Isoleucin.

Das Lacton der Formel IV kann jedoch auch, für den Fall, daß X₁ SO₂Cl ist, zuerst mit einem Amin der Formel VI zu einer Verbindung der Formel VII analog der oben beschriebenen Verfahrensweise umgesetzt werden.
Das so hergestellte Sulfonamidlacton wird dann auf etwa -70 bis -50°C unter Inertgasatmosphäre gekühlt und etwa 1 - 2 Äquivalente, bevorzugt 1,5 - 1,7 Äquivalente einer 2 - 3 molaren, bevorzugt 2,5 molaren DiBAl-Lösung, beispielsweise in Toluol langsam zugegeben. Nach 0,5 bis 2 Stunden bei -70 bis -50°C wird die Verbindung der Formel I bevorzugt extraktiv isoliert und gegebenenfalls durch Umkristallisation gereinigt.

Die Anhydroverbindungen der Formel II können beispielsweise aus Verbindungen der Formel I in denen W Wasserstoff bedeutet, hergestellt werden, beispielsweise durch Zugabe von wasserentziehenden Mitteln wie etwa Thionylchlorid.

Die Verbindungen der Formel I und II reagieren mit razemischen Verbindungen die eine Hydroxy-, Carboxy-, Amino- oder Mercaptogruppe enthalten, mit hoher Selektivität, wodurch eine besonders einfache Reinisolierung eines Enantiomeren ermöglicht wird.

Ein weiterer Gegenstand der Erfindung ist demnach die Verwendung von Verbindungen der Formel I oder II zur Trennung razemischer Verbindungen die eine Hydroxy-, Carboxy,- Amino- oder Mercaptogruppe enthalten.

Besonders geeignet sind die Verbindungen der Formel I und II zur Trennung von razemischen Verbindungen der Formel 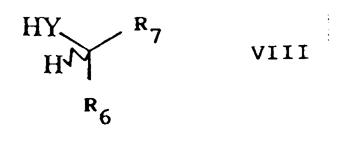
in der Y -O-, -S-, -COO- oder -NH- bedeutet.
R₆ ist dabei bevorzugt eine "sperrige" Gruppe, etwa ein verweigtes oder unverzweigtes Alkyl, bevorzugt mit 1 - 10 C-Atomen, das gegebenenfalls substituiert sein kann, etwa durch Phenyl oder (C₁-C₆)Alkoxy.
Beispiele sind Methyl, Ethyl, n-Propyl, -i-Propyl, sec.-Butyl, t-Butyl oder Hexyl.
R₇ ist bevorzugt eine "planare" oder eine "lineare" Gruppe. Hierzu gehören Aryle, wie Phenyl oder Heteroaryle wie etwa Pyrrolyl oder Pyridinyl die gegebenenfalls durch (C₁ - C₄)Alkyl, (C₁-C₄)Alkoxy oder Hydroxy substituiert sein können, oder -C≡N, -C≡C-, Formyl, Keto, Carbonsäureester oder Amide, die gegebenenfalls substituiert sein können, etwa durch (C₁ -C₄)Alkyl.
R₆ und R₇ können jedoch auch beide aus der planaren Gruppe gewählt werden.

Ein weiterer Gegenstand der Erfindung ist ein Verfahren zur Trennung razemischer Verbindungen die eine Hydroxy-, Carboxy-, Amino- oder Mercaptogruppe enthalten.
Das Verfahren ist in 3 Schritte gegliedert. Zuerst werden eine Verbindung der Formel I oder II und die entsprechende razemische Verbindung in einem geeigneten Verdünnungsmittel gelöst, etwa in wasserfreiem Ether, Dichlormethan, Tetrahydrofuran, Chloroform oder in einer Petrolether/Ether-Mischung (PE/E).
Nach der Zugabe von Triphenylphosphin Hydrobromid (TPHB) wird das Reaktionsgemisch etwa 36 - 60 Stunden, vorzugsweise 48 Stunden bei Raumtemperatur gerührt.
Im 2. Schritt wird das Reaktionsgemisch extraktiv getrennt und als letzter Schritt erfolgt die Hydrolyse des Diastereomerengemisches, indem man das Diastereomerengemisch in einem geeigneten Lösungsmittel löst, auf Rückfluß erhitzt, p-Toluolsulfonsäure zugibt und 3 bis 8 Stunden bei Rückflußtemperatur hält. Anschließend wird Alkalihydrogencarbonat oder Bicarbonat zugegeben und durch Extraktion die reinen Enantiomere gewonnen. Die optische Reinheit kann durch eine Kristallisationsstufe, die vor der Hydrolyse durchgeführt wird, gesteigert werden.

### Beispiel 1: Herstellung des Bromlactols

### (2S-(2alpha,3a-alpha,4beta,7beta,7a-alpha))-7-Brommethyl-8,8-dimethyl-octahydro-4,7-methanobenzofuran-2-ol (n= 1, W = H, X = Br)

Zu einer Mischung aus 20 g (3aR-(3a-alpha,4beta,7beta,7a-alpha)-3a,4,5,6,7,7a-Hexahydro-7,8,8-trimethyl-4,7-methanobenzofuran-2(3H)-on und 1,3 g rotem Phosphor wurden unter Rühren und Eisbadkühlung 16,5 g Brom zugetropft, wobei eine sehr heftige Reaktion einsetzte. Nach Abklingen der Reaktion wurde auf 70°C erhitzt und bei dieser Temperatur nochmals 16,5 g Brom zugegeben. Nach 3 Stunden bei 80°C wurde die Apparatur mit Stickstoff gespült, um entstandenen Bromwasserstoff zu vertreiben. Anschließend wurden 2 ml Wasser zugegeben und das Reaktionsgemisch im Wasserstrahlvakuum bei 135°C so lange abgesaugt, bis keine Gasentwicklung mehr zu beobachten war (etwa 4 Stunden). Anschließend wurde zwischen Dichlormethan und ges. Natriumhydrogencarbonatlösung verteilt, die wäßrige Phase mit Dichlormethan extrahiert und die vereinigten organischen Phasen mit Wasser gewaschen, getrocknet und eingedampft. Das Rohprodukt (26,5 g, 94,2 % d. Th.) wurde aus PE/E umkristallisiert.
Ausbeute: 19,1 g (67,9 % d. Th.) (3aS-(3a-alpha,4beta,7beta,7a-alpha))-Brommethyl-hexahydro-8,8-dimethyl-4,7-methanobenzofuran-2(3H)-on, farblose Kristalle, Schmp. 96 - 100°C, (alpha)_{D}²⁰ = -81,9° (c = 0,68 in Dichlormethan).
-¹H-NMR (CDCl₃): delta = 4,67 (d,J = 8,8 Hz; 1H,H-7a), 3,80/3,68/3, 44/3, 32 (d,2H,CH₂-Br), 1,1-2,6(m, 8H, Aliphaten-H), 1,01/0,8 (2s,6H,2CH₃).-
Zu einer Lösung von 500 mg (3aS-(3a-alpha,4beta,7beta,7a-alpha))-7-Brommethyl-hexahydro-8,8-dimethyl-4,7methanobenzofuran-2(3H)-on in 10 ml wasserfreiem Ether wurden unter Stickstoffatmosphäre bei -20°C 1,8 ml einer 1,2 molaren Lösung von Diisobutylaluminiumhydrid (DiBAl) in Toluol zugetropft. Nach 1 Stunde bei -20°C wurde 1 ml Wasser zugegeben, das Reaktionsgemisch auf 50 ml eiskalte, 10 proz. Essigsäure gegossen und die wäßrige Phase mit Ether extrahiert. Die organische Phase wurde mit halbges. Natriumhyrogencarbonatlösung bis zur Neutralität gewaschen. Die wäßrige Phase wurde mit Ether rückgeschüttelt und die vereinigten organischen Phasen über 5 g triethylaminimprägniertes Kieselgel abgenutscht, getrocknet und eingedampft.
Ausbeute: 491 mg (97,5 % d. Th.) (2S-(2alpha,3a-alpha,4beta,7beta, 7a-alpha))-7-Brommethyl-8,8-dimethyloctahydro-4,7-methanobenzofuran-2-ol, farblose Kristalle, Schmp. 75 - 78°C (aus Petrolether/Ether), (alpha)_{D}²⁰ = -79,6°C (c = 0,22 in Dichlormethan).
-¹H-NMR (CDCl₃): delta = 5,61 (t,J = 5,0 HZ; 1H,2-H), 4,40(d,J=8,1Hz;1H,H-7a), 3,82/3,72/3,45/3,35 (d,2H,CH₂Br), 2,7(bs;1H,OH); 1,1-2,5 (m, 8H, Aliphaten-H); 1, 01/0,88(2s,6H,2CH₃).-

### Beispiel 2: Herstellung des Sulfochlorid-Dilactols

### (2S-(2alpha(2'R*,3'aS*,4'S*,7'R,7'aS*),3a-alpha,4beta,7alpha,7a-alpha))-2,2'-Oxybis((octahydro-8,8-dimethyl-4,7-methanobenzofuran-7-yl)-methansulfonsäure-chlorid) (n = 1, X = SO₂Cl, W = der Rest der Formel Ia)

Zu 1,05 g Essigsäureanhydrid wurden unter Kühlung derart 0,85 ml conc. Schwefelsäure zugetropft, daß die Temperatur nicht über 20°C anstieg. Anschließend wurde 1 g (3aR-(3a-alpha,4beta,7beta,7a-alpha))-3a,4,5,6,7,7a-Hexahydro-7,8,8-trimethyl-4,7-methanobenzofuran-2(3H)-on zugegeben und das Reaktionsgemisch 6 Tage bei Raumtemperatur stehen gelassen. Absaugen und Waschen mit wasserfreiem Ether ergab 600 mg (42,5 % d. Th.) (3aS-(3a-alpha,4beta,7beta,7a-alpha))-8,8-Octahydro-8,8-dimethyl-2-oxo-4,7-methanobenzofuran-7-yl-methansulfonsäure, farblose Kristalle, Schmp. 204°C (aus Essigsäureethylester, unter Zersetzung) (alpha)_{D}²⁰ = -55,1°C (c = 1,34 in Ethanol).
¹H-NMR (DMSO-d₆) nach Schütteln mit D₂O: delta = 4,90 (d,J = 8,4Hz; 1 H,H-7a), 3,03/2,87/2,63/2,47(d,2H,CH₂SO₃), 1,1-1,9 (m,8H, Aliphaten-H), 0,86 (s,6H, 2CH₃).-
100 mg (3aS-(3a-alpha,4beta,7beta,7a-alpha))-8,8-Octahydro-8,8-dimethyl-2-oxo-4,7-methanobenzofuran-7-yl-methansulfonsäure wurden unter Eisbadkühlung und Feuchtigkeitsausschluß mit 77 mg Phosphorpentachlorid verrührt. Nach 8 Stunden bei Raumtemperatur wurde zum öligen Reaktionsgemisch Eiswasser zugegeben, wobei sich ein farbloser Niederschlag bildete. Absaugen und Waschen mit Eiswasser ergab 80 mg (74,9 % d. Th.). (3aS-(3a-alpha,4beta,7beta-7a-alpha))-8,8-Octahydro-8,8-dimethyl-2-oxo-4,7-methanobenzofuran-7-yl-methansulfonsäurechlorid, farblose Kristalle, Schmp. 124 - 127°C (aus Dichlormethan/Ether/Petrolether), (alpha)_{D}²⁰ = -62,6° (c = 1,17 in Essigsäureethylester).
¹H-NMR (CDCl₃): delta = 4,8 - 4,9 (m, 1H,H-7a), 4,36/4,21/3,83/3,68 (d,2H,CH₂-SO₂), 1,1 - 2,9 (m,8H, Aliphaten-H), 1,04/1,03 (2s,6H,2CH₃).-
1,9 g (3aS-(3a-alpha,4beta,7beta,7a-alpha))-8,8-Octahydro-8,8-dimethyl-2-oxo-4,7-methanobenzofuran-7-yl-methansulfonsäurechlorid wurde in ca. 50 ml abs. CH₂Cl₂ gelöst und unter N₂-Atmosphäre auf - 70°C abgekühlt. Danach wurden 4,8 ml einer 1 ,5-molaren DiBAl-Lösung in Toluol zugetropft, sodaß die Reaktionstemperatur -60°C nicht überschritt. Die Mischung wurde eine Stunde bei -70°C gerührt und danach auf ein Gemisch aus 50 ml CH₂Cl₂ und 50 ml 2n HCl geleert. Die beiden Phasen wurden so lange geschüttelt, bis sie durchsichtig wurden, die organische Phase wurde noch einmal mit 2n HCl gewaschen, über Na₂SO₄ getrocknet und zur Trockene eingedampft. Der Schaum wurde in n-Hexan digeriert, wodurch er zu einem farblosen Pulver erstarrte.
Ausbeute: 1,51 g (81 % d. Th.) (2S-(2alpha(2'R*,3'aS*,4'S*, 7'R*,7'aS*),3a-alpha,4beta,7alpha,7a-alpha))-2,2'-Oxybis((octahydro-8,8-dimethyl-4,7-methanobenzofuran-7-yl)-methansulfonsäurechlorid), farbloses Pulver, Schmp: 147 - 152° C (alpha)_{D}²⁰ = -115° (c = 3,26 in CH₂Cl₂), DC: R_{f}: = 0,78 (PE/EE = 3 : 1). -
-¹H-NMR (CDCl₃): delta =5,47 (d,J=2,5Hz; 1H, H-2),4,35 (d,J=12,5Hz; 1H, CHSO₂), 4,30 (d, J= 7,5 Hz; 1H, H-7a), 3,70 (d,J= 12,5 Hz; 1H, CHSO₂), 2,38 (m; 1H, H-3a), 2,07 (m; 2H, H-3), 1,88 (m; 1H, H-6äq), 1,77 (m; 1H, H-4), 1,42 (m; 1H, H-5äq), 1,15 (m; 1H, H-6ax), 1,03/0,91 (2s; 6H, 2CH₃), 0,90 (m; 1H, H-5ax).

### Beispiel 3: Herstellung von Sulfonamid-Lactolen

### Allgemeine Vorschrift zur Synthese der entsprechenden Sulfonamid-Lactone

Einer 5%igen Suspension von (2S-(2alpha(2'R*,3'aS*,4'S*,7'R*,7'aS*),3a-alpha,4beta,7alpha,7a-alpha))-2,2'-Oxybis(octahydro-8,8-dimethyl-4,7-methanobenzofuran-7-yl)-methansulfonsäurechlorid) in abs. Ether wurde so lange abs. CH₂Cl₂ zugesetzt, bis das Produkt in Lösung ging. Anschließend wurden 2-3 Äquiv. der Base zugesetzt und über Nacht gerührt. Der farblose Niederschlag wurde abgesaugt, die Reaktionslösung konzentriert, über Kieselgel filtriert (LM:Ether), mit H₂O gewaschen, die organische Phase über Na₂SO₄ getrocknet und zur Trockene eingedampft. Der Rückstand wurde in n-Hexan/Toluol umkristallisiert.

Beispiel: (n=1, X=SO₂N(C₂H₅)₂): Einer Lösung von 3,0 g (10,2 mmol) (2S-(2alpha(2'R*,3'aS*,4'S*,7'R*,7'aS*),3a-alpha,4beta,7alpha,7a-alpha))-2,2'-Oxybis(octa-hydro-8,8-dimethyl-4,7-methanobenzofuran-7-yl)-methansulfonsäurechlorid wurden 2,66ml (27,7 mmol) Diethylamin zugegeben und das Reaktionsgemisch nach obiger Vorschrift weiterbehandelt.
Ausbeute: 2,4 g (71 % d. Th.) (3aS-(3a-alpha,4beta,7alpha,7a-beta))-N,N-Diethyl-(octahydro-8,8-dimethyl-2-oxo-4,7-methanobenzofuran-7-yl)-methansulfonamid, farblose Kristalle, Schmp. 128 - 130°C, (alpha)_{D}²⁰ = -230° (c = 0,91 in CH₂Cl₂), DC: R_{f} = 0,25 (E/PE = 9:1).
-¹H-NMR (CDCl₃): delta = 4,96 (s; 1H, H-7a), 3,30 (q; 2H, N-CH₂), 3,20 (q; 2H, N-CH₂), 3,25 (d,J=14Hz; 1H, CH-SO₂), 2,76 (d, J=14Hz, 1H, CHSO₂), 2,63-1,33 (m; 9H, Aliphaten-H), 1,22 (t; 6H, CH₂CH₃), 0,97/0,93 (2s; 6H, 2CH₃).-

### Allgemeine Vorschrift für die Reduktion der Sulfonamid-Lactone zu den Sulfonamid-Lactolen

Eine ca. 5 %ige Lösung des jeweiligen, nach obiger Vorschrift hergestellten Sulfonamidlactons, wurde auf -60°C unter N₂-Atmosphäre gekühlt und 1,6 Äquivalente einer 2,5-molaren Diisobutylaluminiumhydridlösung in Toluol langsam zugesetzt. Danach wurde ca. 1 Stunde bei -60°C gerührt, die Reaktionsmischung zwischen CH₂Cl₂/0,25n HCl verteilt und mit CH₂Cl₂ extrahiert. Die organsiche Phase wurde mit NaHCO₃ gewaschen und zur Trockene eingedampft. Der Rückstand wurde in Toluol/n-Hexan umkristallisiert.

Beispiel: (n=1, W=H, X=SO₂N(C₂H₅)₂): 2,24 g (3aS-(3a-alpha,4beta,7alpha,7a-beta))-N,N-Diethyl-(octahydro-8,8-dimethyl-2-oxo-4,7-methanobenzofuran-7yl)-methansulfonamid wurden in 40 ml abs. CH₂Cl₂ gelöst und nach obiger Vorschrift weiterbehandelt.
Ausbeute: 1,90 g (85 % d. Th.) (2S-(2alpha,3a-alpha,4beta,7alpha,7a-alpha))-N,N-Diethyl-(octahydro-2-hydroxy-8,8-dimethyl-4,7-methanobenzofuran-7yl)-methansulfonamid, farblose Kristalle, Schmp.: 129 - 131°C, (alpha)_{D}²⁰ = -211° (c = 1,14 in CH₂Cl₂).
-¹H-NMR (CDCl₃):delta=5,5 (dd; 1H, H-2), 4,59 (d; 1H, H-7a), 3,32 (d,J = 14Hz; 1H,CHSO₂), 3,31 (q; 4H, NCH₂), 2,68 (d,J=14Hz; 1H,CHSO₂), 2,64-1,37(m; 9H; Aliphaten-H,OH), 1,22 (t;6H, CH₂CH₃), 0,97/0,84 (2s;6H,2CH₃).

### Beispiel 4: Herstellung der Sulfonamid-Dilactole

### Allgemeine Vorschrift zur Synthese der Sulfonamid-Dilactole

Einer ca. 5 %igen Lösung von (2S-(2alpha(2'R*,3'aS*,4'S*,7'R*,7'aS*), 3a-alpha,4beta,7alpha,7a-alpha))-2,2'-Oxybis(octahydro-8,8-dimethyl-4,7-methanobenzofuran-7-yl)-methansulfonsäurechlorid) in abs. DMF wurden 2 Äquivalente des entsprechenden Amins (freies Amin oder Hydrochlorid) zugesetzt, die Reaktionsmischung wurde mittels einer Eis-Wasser-Mischung auf ca. 5°C gekühlt und 3 (freies Amin) bzw. 5 (Hydrochlorid) Äquivalente an abs. Triethylamin zugespritzt. Die Reaktionslösung wurde 0,5 bis 2 Stunden bei Reaktionstemperatur bis zur Vervollständigung der Reaktion (DC-Kontrolle) gerührt, danach zwischen Ether und 0,5n HCl verteilt, die org. Phase mit 0,5nHCl 2 - 3 mal gewaschen, über Na₂SO₄ getrocknet und zur Trockene eingedampft. Der Rückstand (überwiegend ein farbloser Schaum) wurde gegebenenfalls mittels Vakuumflashchromatographie gereinigt.

Beispiel:(n=1,X=SO₂(N-Prolylmethylester)):
1g (2S-(2alpha(2'R*,3'aS*,4'S*,7'R*,7'aS*),3a-alpha,4beta,7alpha,7alpha,7a-alpha))-2,2'-Oxybis((octahydro-8,8-dimethyl-4,7-methano-benzofuran-7-yl)-methansulfonsäurechlorid), 1,8 g L-Prolinmethylester Hydrochlorid und 2,2 ml Triethylamin wurden entsprechend obiger, allgemeiner Vorschrift 2 Stunden gerührt.
Ausbeute: 1 g (71 % d. Th.) (2S-(2alpha(2'R*,3'aS*,4'S*,7'R*,7'aS*),3a-alpha,4beta,7alpha,7a-alpha))-2,2'-Oxybis(1-((octahydro-8,8-dimethyl-4,7-methanobenzofuran-7-yl)methylsulfonyl)-L-prolinmethylester), Schmp.: 81-83°C, (alpha)_{D}²⁰=126° (c =0,79 in CH₂Cl₂)
-¹H-NMR (CDCl₃): delta = 5,43 (d;1H,H-2), 4,51 (dd, J₁ =9Hz, J₂=3Hz; 1H, H-alpha), 4,32 (d,J=7,5 Hz; 1H, H-7a), 3,74 (s; 3H, OCH₃), 3,57 (d,J=13Hz; 1H,CHSO₂), 3,55 (m; 2H, H-delta), 3,06(d,J=13Hz; 1H, CHSO₂), 2,3 (m; 1H, H-beta), 2,25 (m; 1H, H-3a), 2,03 (m; 2H,H-3), 1,8-2,0 (m; 3H, H-beta, H-gamma), 1,72 (m; 1H, H-6äq), 1,67 (m; 1H, H-4), 1,40 (m; 1H, H-5äq), 0,88 (m; 1H, H-5ax), 1,07 (m; 1H, H-6ax), 0,98/0,83 (2s; 6H, 2CH₃).

### Allgemeine Vorschrift zur Hydrolyse der Dilactole mit einer Esterfunktion zu den entsprechenden Carbonsäuren:

3 - 5 Äquivalente NaOH wurden in soviel Ethanol gelöst, daß mit dem entsprechenden Ester eine ca. 5 %ige Lösung erhalten wurde. Danach wurde 0,5 bis 2 Stunden unter Rückfluß erhitzt (DC - Kontrolle), die Reaktionslösung zur Trockene eingedampft, in H₂O aufgenommen, zweimal mit Ether gewaschen, mit HCl auf pH = 1 angesäuert und mit Ether extrahiert. Die organische Phase wurde über Na₂SO₂ getrocknet und zur Trockene eingedampft.

Beipiel: (n = 1, X = SO₂(N-Prolin, W = der Rest der Formel Ia) Entsprechend der allgemeinen Hydrolysevorschrift wurden 1 g (2S-(2alpha(2'R*,3'aS*,4'S*,7'R*,7'aS*),3a-alpha,4beta,7alpha,7a-alpha))-2,2'-Oxybis(1-(octahydro-8,8-dimethyl-4,7-methanobenzofuran-7-yl)methylsulfonyl)-L-prolinmethylester) mit 264 mg NaOH umgesetzt.
Ausbeute: 960 mg (quantitativ) (2S-(2alpha(2'R*,3'aS*,4'S*,7'R*,7'aS*), 3a-alpha-4beta,7alpha,7a-alpha))-2,2'-Oxybis(1-((octahydro-8,8-dimethyl-4,7-me thanobenzofuran-7-yl)methylsulfonyl)-L-prolin), farbloser Schaum, Schmp.: 121 - 125°C, (alpha)_{D}²⁰=115° (c= 1,04 in CH₂Cl₂).
-¹H-NMR (CDCl₃): delta = 9,1 (bs; 1H, COOH), 5,38 (s; 1H, H-2), 4,57 (dd,J₁=8Hz, J₂=4,5Hz; 1H, H-alpha), 4,4 (d,J=7Hz; 1H, H-7a), 3,60 (d,J=13Hz; 1H, CHSO₂), 3,5 (m; 2H,H-delta), 3,03 (d,J=13Hz; 1H, CHSO₂), 2,31 (m; 1H, H-3a), 2,21 (m; 1H, H-beta), 1,8 - 2,04 (m; 2H, H-beta, H-gamma), 1,99 (m; 2H, H-3), 1,78 (m; 1H, H-6äq), 1,69 (m; 1H, H-4), 1,45 (m; 1H, H-5äq), 0,90 (m; 1H, H-5ax), 1,04 (m; 1H, H-6ax), 0,98/0,83 (2s; 6H,2CH₃).

### Beispiel 5: Razemattrennung durch Extraktion

Das Verfahren ist in drei Verfahrensschritte gegliedert:
1. Acetalisierung
2. extraktive Trennung und
3. Hydrolyse

### Acetalisierung und extraktive Trennung:

700 mg (2S-(2alpha(2'R*,3'aS*,4'S*,7'R*,7'aS*),3a-alpha,4beta,7a-alpha))-2,2'-Oxybis(1-((octahydro-8,8-dimethyl-4,7-methanobenzofuran-7-yl)methylsulfonyl)-L-prolin) und 1,26 g alpha-(1,1-Dimethylethyl)-benzolmethanol (R₆ = t-Bu, R₇ = Ph,Y = O) wurden in 30 ml abs. Dichlormethan geläst und nach Zusatz von 130 mg an Triphenylphosphin Hydrobromid (TPHB) wurde die Reaktionsmischung 48 Stunden bei Raumtemperatur gerührt. Das Reaktionsgemisch wurde zwischen PE/E = 4 : 1 und einer gesättigten KHCO₃-Lösung verteilt, die wäßrige Phase dreimal mit PE/E = 4 : 1 extrahiert (Ethanol Zusatz zur besseren Phasentrennung), auf ein Zehntel des Volumens eingeengt, mit 2n HCl angesäurert (pH <1) und mit CH₂Cl₂ extrahiert. Die CH₂Cl₂-Phase wurde über Na₂SO₄ getrocknet und zur Trockene eingedampft.
Ausbeute: 1 g (100 %) 1-((2-(2,2-Dimethyl-1-phenylpropoxy)-octahydro-8,8-dimethyl-4,7-methanobenzofuran-7-yl)methylsulfonyl)-L-prolin; Verhältnis (2S-(2alpha(R*),3a-alpha,4beta,7alpha,7a-alpha)) zu (2S-(2a(S*),3a-alpha,4beta,7alpha,7a-alpha)) = 7,2 : 1 (2S-(2alpha(R*),3a-alpha,4beta,7alpha,7a-alpha));farblose Kristalle.
¹H-NMR (CDCl₃): delta: = 4,88 (d,J=4,5Hz; 1H, H-2), 4,55 (dd;1H,H-alpha), 4,28 (s; 1H, H-C*), 3,58 (t; 2H, H-delta), 3,46 (d,J=13,5Hz; 1H,CHSO₂), 3,04 (d,J=13,5Hz; 1H, CHSO₂), 2,52-1,04 (m; 12H, H-3, H-3a, H-4, H-5, H-6, H-beta, H-gamma), 0,87 (s; 9H, C(CH₃)₃), 0,87/0,8 (2s; 6H, 2CH₃).
(2S-(2alpha(S*),3a-alpha,4beta,7alpha,7a-alpha)).
¹H-NMR (CDCl₃): delta = 5,21 (d,J=5Hz; 1H, H-2),4,07 (d,J= 6,5Hz; 1H,H-7a), 4,05 (s; 1H, H-C*), 3,27 (d,J= 14,5Hz; 1H, CHSO₂), 2,95 (d,J=14,5Hz; 1H, CHSO₂), 0,87/0,81 (2s; 6H,2CH₃).
Die PE/E-Phase wurde ebenfalls über Na₂SO₄ getrocknet und zur Trockene eingedampft.
Restalkohol: 865 mg (5,3 mmol; 93 %).

### Hydrolyse:

Das Diastereomerengemisch ((2-(2,2-Dimethyl-1 -phenylpropoxy)-octahydro-8,8-dimethyl-4,7-methanobenzofuran-7-yl)methylsulfonyl)-L-prolin wurde in 30 ml CH₃CN/H₂O = 4 : 1 gelöst, auf Rückfluß erhitzt und 150 mg an p-Toluolsulfonsäure zugegeben. Nach 5 Stunden unter Rückfluß wurde die Reaktionslösung mit NaHCO₃ versetzt, bis fast zur Trockene eingedampft, der Rückstand mit ges. NaHCO₃-Lösung verdünnt und mit Ether extrahiert. Die organische Phase wurde über Na₂SO₄ getrocknet und zur Trockene eingedampft. Abgespaltener alpa-(1,1-Dimethylethyl)-benzolmethanol: 300 mg 195 %); S:R = 6,9 : 1
Die wäßrige Phase wurde mit 2n HCl angesäuert, mit CH₂Cl₂ extrahiert, und nach Zusatz von wenig Toluol über Na₂SO₄ getrocknet und zur Trockene eingedampft.
Rückgewonnenes (2S-(2-alpha(2'R*,3'aS*,4'S*,7'R*,7aS*),3a-alpha,4-beta,7alpha,7a-alpha))-2,2'-Oxybis(1-((octahydro-8,8-dimethyl-4,7-methanobenzofuran-7-yl)methylsulfonyl)-L-prolin): 640 mg (91 %).

### Beispiel 6: Steigerung der optischen Reinheit durch Kristallisation des angereicherten Diastereomerengemisches.

Das Verfahren besteht aus vier Schritten:
1. Acetalisierung
2. Extraktion
3. Kristallisation und
4. Hydrolyse

### Acetalisierung und Extraktion:

6g (2S-(2-alpha(2'R*,3'aS*,4'S*,7'R*,7'aS*), 3a-alpha,4beta,7alpha,7a-alpha))-2,2'-Oxybis(1-((octahydro-8,8-dimethyl-4,7-methanobenzofuran-7-yl)methylsulfonyl)-L-prolin), 10,8 g alpha-(1,1-Dimethylethyl)-benzolmethanol (R₆=t-Bu, R₇=Ph, Y=O) und 550 mg TPHB wurden 4 Tage in CCl₄ bei Raumtemperatur gerührt, das Reaktionsgemisch, nach Zugabe von NaHCO₃, zur Trockene eingedampft und in einer gesättigten Lösung von KHCO₃ aufgenommen. Die basische Lösung wurde mit PE/E unter Zusatz von Ethanol zur Phasentrennung extrahiert, die organische Phase zur Trockene eingedampft, wiederum in Ether aufgenommen, über Na₂SO₄ getrocknet und zur Trockene eingedampft. Restalkohol: 8,1 g (100 %).

Die wäßrige Phase wurde eingeengt, mit 2n HCl angesäuert (pH< 1) und mit CH₂Cl₂ extrahiert. Die organische Phase wurde über Na2SO4 getrocknet und zur Trockene eingedampft.
Ausbeute: 7,9 g (95 %); Verhältnis (2S-(2alpha(R*), 3a-alpha, 4beta, 7alpha, 7a-alpha) zu (2S-(2alpha(S*),3a-alpha,4beta,7alpha,7a-alpha)) = 10 : 1

### Kristallisation:

Das Diastereomerengemisch der Acetale wurde aus Acetonitril umkristallisiert. Die ausgeschiedenen Kristalle wurden abfiltriert und im Wasserstrahlvakuum bei 40°C getrocknet.
Ausbeute: 5,6 g (68 %) reines Diastereomer (2S-(2alpha(R*),3a-alpha,4beta,7alpha,7a-alpha)-((2-(2,2-Dimethyl-1-phenylpropoxy)-octahydro-8,8-dimethyl-4,7-methanobenzofuran-7-yl)methylsulfonyl)-L-prolin; de > 99 %.

### Hydrolyse:

Die Hydrolyse des reinen Diastereomeren (2S-(2alpha(R*), 3a-alpha, 4beta, 7alpha, 7a-alpha))-((2-(2,2-Dimethyl-1-phenylpropoxy)-octahydro-8,8-dimethyl-4,7-methanobenzofuran-7-yl)methylsulfonyl)-L-prolin erfolgte analog zu obiger Vorschrift.
Ausbeute: 3,9 g (65 %) (2S-(2alpha(2'R*,3'aS*,4'S*,7'R*,7'aS*),3a-alpha,4beta,7alpha,7a-alpha))-2,2'-Oxybis(1-((octahydro-8,8-dimethyl-4,7-methanobenzofuran-7-yl)methylsulfonyl)-L-prolin) und 1,7 g (65 %) S-alpha-(1,1-Dimethylethyl)-benzolmethanol; ee > 99 %.

Analog Beispiel 5 bzw. 6 wurden weitere Razemattrennungsbeispiele mit verschiedenen Verbindungen der Formel I durchgeführt.

Weiters wurde ein Vergleichsversuch mit einer Verbindung aus US 4 497 960, die einer Verbindung der Formel I der vorliegenden Erfindung mit X = Wasserstoff entspricht, durchgeführt.

Die Ergebnisse der Versuche für verschiedene razemische Verbindungen der Formel 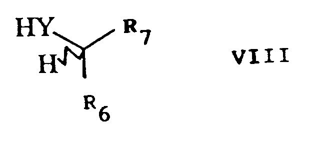
in der Y -O- bedeutet, sind in Tabelle 1 zusammengefaßt.

Die Nummerierung entspricht dabei der Nummerierung der besonders bevorzugten Verbindungen von Seite 3 - 5.

Die Verhältnisse geben jeweils das Verhältnis von (R)-Form : (S)-Form an.

**Tabelle 1**

| | X | t-Bu/Ph | R₆/R₇ Me/Ph | Me/CN | m-PPh/CN |
|---|---|---|---|---|---|
| Vergl. | H | 5,00:1 | 2,40:1 | 1,63:1 | 1,60:1 |
| 1 | Br | | 3,20:1 | 2,60:1 | |
| 4 | SO₂NMe₂ | 4,20:1 | 3,10:1 | | |
| 5 | SO₂NEt₂ | 6,27:1 | 3,60:1 | 4,20:1 | 2,70:1 |
| 6 | SO₂NBn₂ | 7,80:1 | 3,50:1 | 4,70:1 | 3,60:1 |
| 7 | SO₂N(Bn)CH₂COOH | 10,00:1 | 4,00:1 | 3,80:1 | 3,60:1 |
| 8 | SO₂(L-Prolinmethylester) | 11,00:1 | 3,80:1 | 5,00:1 | 2,50:1 |
| 9 | SO₂(L-Prolin) | 7,75:1 | 3,50:1 | 2,70:1 | 2,80:1 |
| 10 | SO₂(D-Prolinmethylester) | 9,00:1 | 3,00:1 | 3,00:1 | |
| 11 | SO₂(D-Prolin) | 14,00:1 | 3,50:1 | 3,40:1 | |
| 12 | SO₂N(CH₂COOMe)₂ | 11,00:1 | 4,30:1 | 4,10:1 | 2,70:1 |
| 13 | SO₂N(CH₂COOH)₂ | 7,70:1 | 4,40:1 | 2,90:1 | |
| 14 | SO₂NHCH₂CH₂COOMe | 4,90:1 | | | |
| 15 | SO₂NHCH₂CH₂COOH | 5,60:1 | 2,90:1 | 2,90:1 | |
| 16 | SO₂N(Bn)CH₂CH₂COOMe | 8,00:1 | 5,00:1 | 5,00:1 | |
| 17 | SO₂N(Bn)CH₂CH₂COOH | 9,00:1 | 3,10:1 | 3,10:1 | |
| 18 | SO₂N(CH₂CONHBn)₂ | 4,90:1 | 3,10:1 | 1,30:1 | 1,10:1 |
| 19 | SO₂(L-Di-Prolinmethylester) | 11,00:1 | 3,00:1 | 5,00:1 | 3,70:1 |
| t-Bu tertiär-Butyl Ph Phenyl Me Methyl m-PPh meta-Phenoxy-phenyl Bn Benzyl | | | | | |

## Patentansprüche

1. (R)- und (S)-Enantiomere der Formel 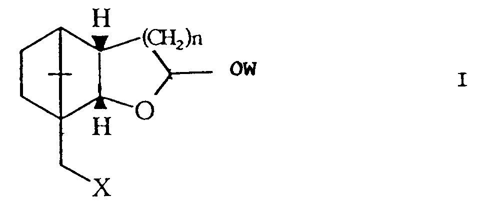 in der n 1 oder 2,
W Wasserstoff, Alkyl, Cycloalkyl oder den Rest 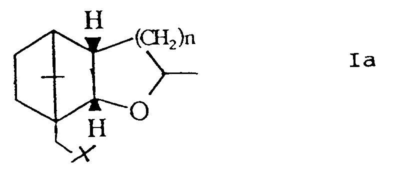 bedeuten, sowie für den Fall, daß W Wasserstoff ist, deren Anhydroverbindungen der Formel 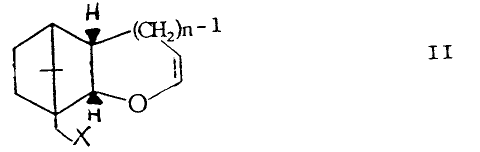 in der n wie oben definiert ist,
dadurch gekennzeichnet, daß X Halogen, SO₃H, SO₂Cl oder SO₂NR₁R₂ bedeutet, wobei R₁ und R₂ unabhängig voneinander Wasserstoff, substituiertes oder unsubstituiertes, verzweigtes oder unverzweigtes Alkyl, substituiertes oder unsubstituiertes Aryl oder Heteroaryl bedeuten, oder R₁ und R₂ gemeinsam mit dem Stickstoff einen substituierten oder unsubstituierten Heterocyclus bilden.

2. (R)- und (S)-Enantiomere und deren Anhydroverbindungen nach Anspruch 1, dadurch gekennzeichnet, daß W Wasserstoff oder den Rest der Formel Ia und X die Gruppe SO₂NR₁R₂ bedeuten, in der R₁ und R₂ unabhängig voneinander Wasserstoff, unverzweigtes oder verzweigtes (C₁ - C₁₀)Alkyl, das gegebenenfalls durch Phenyl, COOR₃, oder CONR₄R₅ substituiert sein kann, bedeuten, wobei R₃ Wasserstoff oder (C₁ - C₆)Alkyl und R₄ und R₅ unabhängig voneinander Wasserstoff, (C₁ - C₆)Alkyl oder Benzyl sein können, oder R₁ und R₂ gemeinsam mit dem Stickstoff einen 3 - 6 gliedrigen Heterocyclus bilden, der gegebenenfalls mit COOR₃ substituiert sein kann, wobei R₃ die oben angegebene Bedeutung hat und n wie in Anspruch 1 definiert ist.

3. Verfahren zur Herstellung von Verbindungen der Formel I nach Anspruch 1, dadurch gekennzeichnet, daß
a) ein Lacton der Formel 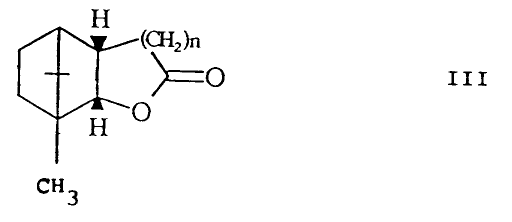 in der n wie in Anspruch 1 definiert ist, mit einem Halogenierungsmittel oder mit Schwefelsäure und gegebenenfalls anschließender Überführung in das Sulfochlorid zu einem Lacton der Formel 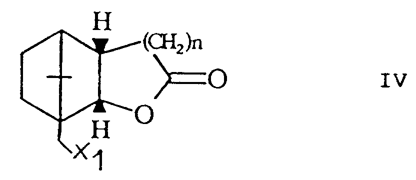 in der X₁ Halogen, SO₃H oder SO₂Cl bedeutet und n wie oben definiert ist,
umgesetzt wird, worauf
b) das Lacton der Formel IV zu einer Verbindung der Formel V 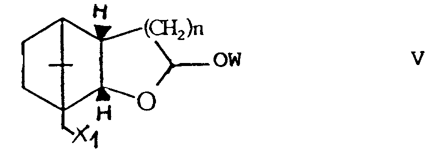 in der W wie in Anspruch 1 definiert ist,
reduziert wird und
c) gegebenenfalls eine Verbindung der Formel V für den Fall, daß X₁ die Gruppe SO₂Cl bedeutet mit einem Amin der Formel
NHR₁ R₂ VI
in der R₁ und R₂ wie in Anspruch 1 definiert sind, zu einer Verbindung der Formel I umgesetzt wird oder
d) das Lacton der Formel IV für den Fall, daß X₁ die Gruppe SO₂Cl bedeutet, gegebenenfalls zuerst mit einem Amin der Formel VI zu einer Verbindung der Formel 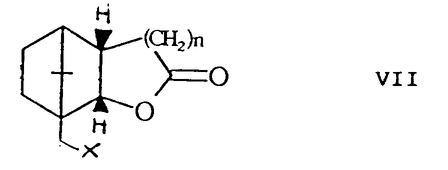 in der X wie in Anspruch 1 definiert ist,
umgesetzt wird und anschließend
e) die Verbindung der Formel VII zu einer Verbindung der Formel I reduziert wird.

4. Verwendung der Verbindungen der Formel I und II zur Trennung razemischer Verbindungen, die eine Hydroxy-, Carboxy-, Amino- oder Mercaptogruppe enthalten.

5. Verwendung der Verbindungen der Formel I und II zur Trennung razemischer Verbindungen der Formel VIII 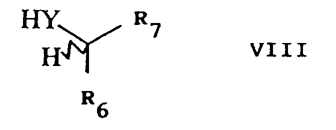 worin Y -O-, -S-, -NH-, -OOC-,
R₆ substituiertes oder unsubstituiertes, verzweigtes oder unverzweigtes Alkyl,
R₇ substituiertes oder unsubstituiertes Aryl oder Heteroaryl, -C≡N, -C≡C-, Formyl, Keto, einen Esterrest oder einen unsubstituierten oder substituierten Amidrest bedeuten.

6. Verfahren zur Razemattrennung, dadurch gekennzeichnet, daß eine Verbindung der Formel I oder II nach Anspruch 1 mit einer razemischen Verbindung, die eine Hydroxy-, Carboxy-, Amino- oder Mercaptogruppe enthält umgesetzt wird, das bevorzugt gebildete Diastereomere vorzugsweise durch Kristallisation oder Extraktion abgetrennt und durch Hydrolyse in das reine Enantiomere überführt wird.
